# EUROPEAN PATENT APPLICATION

(11) **EP 2 039 379 A1**
(43) Date of publication of application: **25.03.2009**
(21) Application number: 08165036.8
(22) Date of filing: 24.09.2008
(51) Int. Cl.: A61M 1/00

(54) **Shutoff valve mechanism for suction devices**

(30) Priority: 24.09.2007 US 903829; 19.09.2008 US 284179
(71) Applicant: Huddleston, Herbert, Encino, CA 91316 (US)
(72) Inventor: Huddleston, Herbert, Encino, CA 91316 (US)
(74) Representative: Fenlon, Christine Lesley

(57) **Abstract**

A disposable shutoff valve apparatus (210) used in conjunction with a surgical suction device (204) having a suction nozzle (205) for stopping the suction action and muffling the noise from the suction device (204) during surgery. The apparatus (210) includes an upper portion (212), a base portion and a flexible member (216). The base portion can be securely attached to a surgical drape around a patient during surgery or other stationary object. The suction nozzle (205) of the suction device (204) can be inserted into and through the open end (212) of the suction chamber (218) and passes through the upper retaining member such that the suction nozzle (205) pulls the flexible member (216) toward itself, and thereby the flexible member (216) stops the suction action of the suction nozzle (205) and muffles the noise from the suction nozzle (205) of the suction device (204) and further simultaneously grips the suction device (204) within the suction chamber (218).

## Description

### BACKGROUND OF THE INVENTION

### 1. Field of the Invention

The present invention relates generally to the field of surgical instruments. More particularly, the present invention relates to the field of surgical instrument apparatuses for holding and cleaning a plurality of surgical instruments used together in a surgery, such as a cautery, a suction device or the like, when not in use and for holding the tube and/or cord attached to the surgical instrument in a predetermined location but permitting movement in response to the surgeon pulling the surgical instrument. In particular, the present invention relates to the field of medical shutoff valves used in conjunction with surgical suction devices for easily shutting off and muffling the noise from the suction nozzle when not in use and for gripping the suction device to secure it in a fixed location during the operation.

### 2. Description of the Prior Art

A flexible suction hose is usually used for evacuating blood or other fluids from the surgical wound-cavity during operations on humans or animals. One end of the suction hose is attached to a continuous negative-pressure source located a few feet away from the operating table and sterile operating field. The surgeon's end of the sterile hose is usually fitted with a rigid suction nozzle which is available in different sizes and shapes.

The suction hose is usually anchored to a convenient location on the sterile drapes, such that a loop of the suction hose is formed between the anchorage and the nozzle. The length of the loop of the hose can be adjusted to allow easy reach of the suction tip to all parts of the wound. The hose is usually anchored by wrapping two folds of the sterile drapes around the hose and clamping the folds together with a stainless steel surgical clamp (i.e., Alice clamp, towel clip and etc.). Alternatively, the surgical clamp is directly attached to the drapes and the hose is threaded through one or both finger holes of the clamp handles.

When the suction nozzle is not in use, the suction nozzle is placed on the drapes over the patient, or is placed in a scabbard comprised of an elongated metal or plastic tube container which is closed at one end, or a flat, soft-plastic pocket or sheath, either of which is attached to the sterile drapes by means of a surgical clamp or by adhesive backing.

Suction is provided continuously precluding the need for the surgeon to actuate valves, operate switches and/or the like.

This overall prior art arrangement has many disadvantages. The upper surfaces of the drapes are irregularly convex surfaces and do not provide a secure storage space. The loop of the hose and its nozzle, and other instruments, such as the cautery and its cable, that are placed on this surface have a constant gravitational tendency to slide off onto the floor where they become contaminated and have to be replaced.

Further, although the surgeon may set the loop of tubing and its nozzle down at a point on the drapes that seems secure, the hose is unruly and does not readily remain where it is placed. The hose has a tendency to spring to a different location determined by the stiffness, springiness and other physical characteristics of plastic hose, as well as by the nature and orientation of the hose anchorage. It may thus spring to a less secure location from where it may fall to the floor or it may spring to a location where the surgeon may not readily locate it without taking his or her eyes off the operation.
A hard plastic or metal scabbard, if used is usually anchored to the sterile drapes with a towel clip or other clamp passed through a hole or loop near its upper end. The towel clip often doubles as an anchorage for the vacuum hose by threading the hose through the clamp's finger holes. This provides no control over the direction in which the anchored hose will lie. Also, the nozzle is frequently sprung from the scabbard by the springy properties of the loop of plastic tubing. Also, being anchored only at its upper end, the scabbard is unstable and prone to being upended by the weight of the tubing, thereby causing its contents to be dumped onto the floor. Also, when the nozzle is in the scabbard, a stiff loop of hose between the nozzle and anchor site often protrudes vertically well above the scabbard, where it may get contaminated against the surgeon's mask or otherwise obtrude into the surgeons work space.

If a towel clip or other clamp-like instrument is used to anchor the scabbard or the suction tubing, it can penetrate or tear the sterile drapes. A plastic pocket with a pressure-sensitive adhesive backing is commercially available for housing the suction nozzle, but its adhesive usually does not stick well to the drapes, it is usually too shallow and inserting the nozzle into a flat, collapsed pocket can be somewhat cumbersome.

The surgeon and his team are thus constantly distracted by concern over the hose and nozzle sliding off onto the floor or obtruding into the surgical area or by having to locate the nozzle when needed.

If the hose or nozzle does fall to the floor, the operation has to be interrupted while it is replaced, unnecessarily prolonging the operating and anesthesia time. An attendant may have to leave the operating room to find replacements. The tubing has to be detached from its anchorage and from the vacuum source, and the new tubing and nozzle have to be connected and anchored. The attachment and re-attachment of the anchoring clamp increases the risk of tearing the drapes and contaminating the sterile field. The lost time and the cost of replaced instruments are additional to the cost of the operation.

The provision of continuous suction without the ability to easily shut-off when not in use has several disadvantages. The suction nozzle creates a continuous, objectionable hissing noise which is distracting and makes for an uncomfortable workplace. Any hard, tube-like scabbard usually amplifies the objectionable sound. Operating room personnel frequently stop the noise by folding the suction hose on itself into a tight loop, thereby closing off the lumen of the hose and jamming the loop into any available suitable space. Alternately, a surgical clamp (hemostat, etc.) is used to clamp off the tubing.

Either method stops the hissing sound, but it takes a two-handed technique to remove the clamp, refold the tube and re-apply the clamp. Also, a clamp adds weight to the tubing rendering it even more likely to upend an unstable scabbard. Suction nozzles are available with a finger-operated on-off valve, but they are inconvenient and are almost never used.

Another problem with prior art is the residual bacteria in the room air of even the cleanest of operating rooms. The continuous negative pressure of the suction hose, draws a constant flow of room air to and through the tip of the nozzle. The nozzle is frequently dipped into, and it is therefore constantly coated by, blood and other body fluids, which form a sticky bacterial culture medium on the nozzle tip to which bacteria from the constantly flowing air can adhere. These bacteria can be a source of wound infection, either directly when the suction nozzle is reintroduced into the wound or, indirectly when the suction nozzle is housed in the same container as other instruments and comes into direct contact with such other instruments. Having means to easily shut off the suction nozzle when it is not in use would thus decrease the chances of wound infections.

Another problem with prior art is that the suction hose is usually connected via a series of canisters to other suction hoses in use in the same operating room, either to additional suction lines used by the surgeon or to a separate suction line used by the anesthesiologist for suction secretions from the patient's throat. When multiple suction hoses are in use in the same operating room, they siphon off negative vacuum pressure from each other, mutually decreasing the suction efficiency of all the lines in use.

The vacuum lines from each operating room in a suite of multiple operating rooms are usually interconnected via a central vacuum pipe connected to a central vacuum pump. The negative pressure lost through any open vacuum hose reduces the strength of the vacuum to other vacuum hoses in the same operating room or in other operating rooms fed by the same system. Occluding suction hoses that are not in actual use therefore increases the general efficiency of the suction system in the entire operating room suite.

A further problem with prior art devices is that the tip of the suction nozzle frequently becomes clogged with soft tissue or bone fragments. The surgeon has to pry the blockage with a long narrow needle-like instrument to dislodge the blockage. Most often the tip of the cautery is used, but it is usually too short or too thin, and the tine of a hemostat is usually too thick, short and curved for dislodging the blockage. In addition, both methods require two hands to perform the dislodging maneuver.

The pencil-like cautery and its flexible cable share many of the problems encountered with the suction hose and its suction nozzle. Its cable must have anchorage, and the device and its loop of cable are also often laid on the drapes over the patient, thereby having a similar tendency to fall to the floor. Many suppliers package it with a small hard-plastic scabbard which is clamped to the drapes with a towel clip, rendering the scabbard unstable as noted above.

A flat soft plastic pocket is commercially available. It is secured to the drapes by a pressure-sensitive adhesive. The main disadvantage is that its thin wall and hence the underlying drapes are susceptible to penetration by the sharp tip of the cautery, with the potential for contamination or injury to the patient's underlying skin by sharp penetration, electric or thermal injury. The pocket is usually in a collapsed state which can make insertion of an instrument cumbersome.

Frequently, the scabbards used for the suction nozzle doubles as a holder for the cautery, where the suction hose and cautery line frequently become entangled. The close proximity of the two instruments increases the risk of bacterial cross-contamination and the combined weight adds to the tendency for the scabbard to upend, dropping both the instruments on the floor. For these reasons it is desirable to have separate holders for the cautery and the suction nozzle.

The cautery has an additional problem in that its flat, paddle-like metal electrode often becomes caked with charred tissue rendering it less conductive and therefore less efficient. A small swatch of abrasive paper is commercially available for cleaning the tip. It is applied to the surgical drapes by means of an adhesive backing. These swatches have the disadvantage of being flat so that only the tip of the electrode can be cleaned unless the surgeon takes the two-handed method of bending the electrode to an angle to present a flat surface parallel the flat swatch and then having to bend the electrode in the opposite direction to clean its other side in a similar manner and then having to straighten out the bent electrode.

Surgeons repeatedly use many other surgical instruments. These surgical instruments are often placed on the irregular upper surfaces of the drapes which cover the patient. Some of these surgical instruments are expensive and fragile and some have attached fiber-optic or electrical cables. Therefore, some means is required for securing and reliably retaining these instruments, as well as for anchorage their fiber-optic cables or other extensions.

United States Patent No. 3,128,072 issued to Shibata on April 7, 1964 discloses an article attaching device which includes a back member. The back member comprises a film of a flexible synthetic resin and a back surface which is coated with an adhesive agent. The adhesive surface is applied with an easily removable separator such as paper coated with a parting agent or cellophane.

United States Patent No. 3,696,920 issued to Lahay on October 10, 1972 discloses a device for organizing objects. It comprises a block of a semi-rigid foam which has a plurality of channels of a configuration adapted to retain the object therein, a beveled slot providing communication between the surface of the block and the channel, the width of the slot narrowing as it approaches the channel, and means for adhesively securing an outer surface of the block to a suitable supporting surface. The object is inserted through the beveled slot into the channel where it is retained in a locked position until needed. The object is then removed for use from the channel by expanding the slot sufficiently to permit the object to be withdrawn from the channel through the slot. The device only anchors tubes, cables or cords and provides no directionality to the secured object.

United States Patent No. 4,074,397 issued to Rosin on February 21, 1978 discloses a disposable device for securing cords, tubes, and the like during surgical or other medical operations. The device may be fastened to the paper or fabric sheet which covers the patient during surgery. The device comprises a thin, flexible pad that has a pressure-sensitive adhesive layer on one side so that it may be removably attached to the aforesaid sheet. It also has an elongated flexible strip portion integral with the pad. The strip portion is wrapped around the cord or tube to be secured by the device and anchored by a VELCRO®. The device only anchors tubes, cables or cords and provides no directionality to the secured object.

United States Patent No. 4,174,816 issued to Olson on November 20, 1979 discloses a sterile surgical cord and tube retractor. The device includes a housing adapted to be supported on the instrument table positioned adjacent the surgical filed. A plurality of spring-tensioned retractors within the housing separately hold lengths of tubing and cord, permitting them to be withdrawn from the housing for use and then retracted back into the housing.

United States Patent No. 4,417,710 issued to Adair on November 29, 1983 discloses a combined surgical instrument and tube holder device. The device is provided for yieldably supporting a hose and/or cord extending from a surgical instrument. The device includes a pad which is adhesively securable to a surgical drape or other surface and is connected to a releasable hose holding means by a stretchable member. The hose holding means includes a strip having a foam layer on one side and a fabric layer of intertwining material on the other side and a tab attached to one end of the strip and having an interlocking surface which releasably adheres to the fabric layer so as to hold the hose and/or cord in desired location while allowing them to move in response to movement of the surgical instrument. In one embodiment, the outer side of the pad has a layer of intertwining material and a strip of interlacing material is adhesively attached to the surgical instrument so that the instrument can be nested on the pad by pressing the interlacing material against the intertwining material on the pad.

United States Patent No. 4,793,483 issued to Holmes on December 27, 1988 discloses a tray for surgical patties. The tray is made of metal and is held to the drapes by means of alligator clips. Holders on the outer edges of the tray are provided for holding an electric cautery, cutter and forceps.

United States Patent No. 5,102,399 issued to Chu on April 7, 1992 discloses a clinical tube holder valve assembly and method. The holder assembly has a tube-receiving passage attached to a mounting block and a pressure-sensitive adhesive thereon for selectively mounting the suction tube holder. A portion of the fluid-flow tube is selectively folded on itself and inserted into the tube-receiving passage for being held therein at a fixed location with a blocked lumen.

United States Patent No. 5,160,106 issued to Monick on November 3, 1992 discloses an adaptor for anesthesia equipment. The apparatus comprises a support member for suction tubing and a catheter, means for clamping the support member to the operating room table and a passageway through the support member for receiving one end of the suction tubing. A catheter is provided which has one end for insertion in the patient's mouth and the other end for connection to one end of the suction tubing. Connection means is provided with one end of the suction tubing for preventing the catheter from passing through the passageway. The passageway is constructed and arranged so that the tubing can be pulled up through the support member to permit the catheter to reach the mouth of the patient and when released will slide back down and stop at the catheter so as to be readily available for reuse. A clamp is carried by the support member for clamping and unclamping the suction tubing and for controlling the suction through the suction tubing to the catheter. The support member has a portion which is shaped to receive a bar on the operating room table and means for clamping the support member to the operating room table. The clamping means comprises a screw member threadedly carried by the support member and one end adapted to engage the bar. The passageway through the support member includes a wall structure which provides low friction with respect to the suction tubing to be pulled therethrough. The passageway through the support member includes a tubular sleeve which has an inner surface for providing low friction with respect to the suction tubing when pulled therethrough.

United States Patent No. 5,334,186 issued to Alexander on August 2, 1994 discloses medical tubing and implement organizer. It allows medical implements to be held in a convenient location proximate to a patient and also allows the medical tubes to be organized and ordered according to size. The tubes are in generally cylindrical lateral bores.

United States Patent No. 5,533,618 issued to Pickels, Jr. on July 9, 1996 discloses a surgical holster for organizing hoses. The hoses are held in generally cylindrical lateral bores in the tubing holder. The hose holding portion of the device is demountably attached to the flat base portion.

United States Patent No. 6,431,500 issued to Jacobs et al. on August 13, 2002 discloses a flexible tube or cord anchoring apparatus which includes a base and a securement member. A cover is attachable at the base and includes a raised shield section which has an access opening. A mounting material layer is affixed at an opposite surface of the base for selected location and securement of the apparatus. In use, the hose or cord is releasably receivable at the securement member through the access opening at the shield section of the cover, where the securement member and the hose or cord received therein being recessed relative to the shield section.

United States Patent No. 6,575,298 issued to McArthur et al. on June 10, 2003 discloses a surgical instrument holder which includes a holder body with connecting adjacent elongated cylinders. The cylinders are able to hold a plurality of surgical instruments such as a diathermy pencil and suction means, or two laparoscopic instruments or the like, and allow easy removal of the instruments.
United States Patent No. 3,982,357 issued to Eldridge et al. on September 28, 1976 discloses a cleaning device for cautery. It includes a supporting frame adapted to be attached to a towel or drape used in surgery and held by an atraumatic clip. The frame has a pair of abrasive strips having mutually engaging surfaces provided with diverging entrance ends for a cauterizing knife to be inserted thereto.

United States Patent No. 6,021,540 issued to Miller et al. on February 8, 2000 discloses a tip cleaner for operating room instruments. It includes a base, upstanding bristles, at least one sharp vertical edge and a flat top.

United States Patent No. 5,228,851 issued to Burton on July 20, 1993 discloses a single-use disposable prophylactic elastic sleeve adapted to be readily placed on a handle of a dental or medical instrument to prevent the transmission of bacteria from one patient to another. The sleeve is provided with finger engaging ring-shaped retention members to aid in placing and expanding the sleeve from a collapsed position over the handle to expose only the patient engaging portion of the instrument.

United States Patent No. 5,441,410 issued to Segerdal on August 15, 1995 discloses a disposable saliva ejector that has a formable hollow tube with a tip on the end with openings for drawing in saliva.

United States Patent No. 5,464,397 issued to Power Jr. on November 7, 1995 discloses a bacteria valve for preventing backflow of bacteria. The valve includes a chamber and a tubular member positionable in the chamber having one or more reversely lipped fins. The chamber and tubular member provide a tortuous path that effectively limits backflow of bacteria and other unhealthy substances.

United States Patent No. 7,131,839 issued to March on November 7, 2006 discloses backflow prevention sleeve for suction devices. It includes a sleeve for preventing backflow and cross-contamination between patients of fluid and particulate matter. The sleeve includes a sleeve inlet end that sealingly attaches to and surrounds at least a portion of the suction device of the vacuum hose to the evacuator tip.

It is highly desirable to have a very efficient and also very effective design and construction of a disposable surgical holder and cleaner apparatus which can securely retain surgical instruments when not in use during surgery, but allow easy removal of surgical instruments when they are required during surgery. In addition, the apparatus may also provide means for easily shutting off the suction nozzle when not in use, de-clogging the tip of the suction nozzle as needed and scraping char off the tip of the cautery instrument, all with a one-handed technique.

It is further desirable to have a very efficient and also very effective design and construction of a disposable shutoff valve means for easily shutting off and muffling the noise from the suction nozzle of a suction device when not in use during surgery.

### SUMMARY OF THE INVENTION

A disposable surgical instrument holder and cleaner apparatus is disclosed which can securely retain a plurality of surgical instruments, such as a suction device, a cautery and other instruments when not in use during surgery. A secure, sterile, lightweight, flame-retardant, non-conducting, non-toxic device is provided, which converts the generally irregular, generally convex, unusable, unstable surface over a draped surgical patient into usable, stable, working space.

The surgical instrument apparatus comprises a generally rectangular shaped body made from a semi-rigid foam or other suitable material which has one or more elongated cylinders for retaining a plurality of surgical instruments when not in use during surgery. The foam walls of the apparatus provide physical, electrical and thermal insulation from the patient. One of the elongated cylinders has means for shutting off the suction nozzle of the suction device when not in use. The means includes a sealed airtight chamber with a passageway which decreases in diameter to accommodate different sizes of suction nozzle, for gripping the suction nozzle, and shutting off the flow of negative pressure vacuum when the suction nozzle is not in use. One or more elongated slotted channels are respectively associated with the elongated cylinders, for retaining hoses and electrical wires of the surgical instruments. These slotted channels are angled to force direction on the hoses and electrical wires of the surgical instruments. The rectangular body of the apparatus has a conformed bottom surface for conforming to an irregular support surface. The apparatus further has an elongated cleaning spike or pin for de-clogging the suction nozzle of the suction device. The apparatus further has an abrasive pad for scraping char off the tip of the cautery.

An alternative shut-off means can be accomplished with provision of conical cavity, wide at its mouth and progressively narrower so that a narrow nozzle will find anchorage at a deep level and a wider nozzle will meet resistance and anchorage at a lesser depth. Second means is by the provision of two or more diaphragms located along the length of the cylinder. Each diaphragm has a hole for the passage of the suction nozzle and progressively decreases in diameter to accommodate different sizes of suction nozzle of the suction device.

It is desirable to provide a surgical instrument apparatus that overcomes the problems of the prior art but allows easy removal of a plurality of surgical instruments for surgical procedures.

It is also desirable to provide a surgical instrument apparatus which can securely hold the surgical instruments used in surgery that are needed to be in close proximity to the surgeon, some of which surgical instruments are attached to a fiber-optic cable, hose or electrical cord.

It is also desirable to provide a surgical instrument apparatus that is disposable.

Further, it is desirable to provide a surgical instrument apparatus that has means for accommodating different diameters of a suction nozzle of a suction device and provides secure housing for the suction nozzle of the suction device, the cautery and other surgical instruments.

It is also desirable to provide a surgical instrument apparatus that has means for easily shutting off the suction nozzle of the suction device when not in use.

It is a further desirable to provide a surgical instrument apparatus that has means for de-clogging the tip of the suction nozzle as needed. It is also desirable to provide a surgical instrument apparatus that has means for scraping char off the tip of the cautery.

It is still further desirable to provide a surgical instrument apparatus that conforms to the irregular, generally convex surface of a patient's body converting it into a flat or concave surface for securely holding a plurality of surgical instruments.

An embodiment of the present invention is a disposable shutoff valve apparatus used in conjunction with suction devices for shutting off and muffling the noise from suction nozzles when not in use during surgery.

It is desirable to provide a shutoff valve means used in conjunction with a suction device that is disposable.

It is also desirable to provide a shutoff valve means that allows a surgeon or the like to shutoff the suction device without start, stop, and restart the suction device during surgery.

It is also desirable to provide a shutoff valve means that allows a surgeon or the like to muffle the noise from the suction device without start, stop, and restart the suction device during surgery.

It is also desirable to provide a shutoff valve means that does not require any additional equipment, but can be used with a practitioner's normal suction equipment.

It is also desirable to provide a shutoff valve means that easily stops the suction device when it is not in use and thus decrease the chances of wound infections.

It is also desirable to provide means for gripping the suction nozzle so that it remains securely anchored in a fixed location during surgery.

### BRIEF DESCRIPTION OF THE DRAWINGS

Referring particularly to the drawings for the purpose of illustration only and not limitation, there is illustrated:
FIG. 1 is a perspective view of disposable surgical instrument apparatus for cleaning and holding surgical instruments, hoses and electrical cords;
FIG. 2 is an enlarged front end plan view of the disposable surgical instrument apparatus shown in FIG. 1;
FIG. 3 is a cross-sectional view taken along line 3-3 of FIG. 2;
FIG. 4 is a cross-sectional view taken along line 4-4 of FIG. 2;
FIG. 5 is a cross-sectional view taken along line 5-5 of FIG. 2;
FIG. 6 is a cross-sectional view taken along line 6-6 of FIG. 2;
FIG. 7 is a cross-sectional view taken along line 7-7 of FIG. 2;
FIG. 8 is a top perspective view of the disposable surgical instrument apparatus shown in FIG. 1;
FIG. 9A is a perspective view of a cleaning spike;
FIG. 9B is a perspective view of another cleaning spike;
FIG. 10A is a perspective view of a diaphragm;
FIG. 10B is an alternative embodiment of FIG. 4 with the diaphragm inserted within the suction cylinder;
FIG. 11 is a perspective view of the disposable surgical instrument apparatus attached to a surgical drape across a patient thereon;
FIG. 12 is a partial perspective view of an abrasive pad attached to the holder body of the disposable surgical instrument apparatus;
FIG. 13 is a cross-sectional view taken along line 13-13 of FIG. 12;
FIG. 14 shows an alternative construction of the disposable surgical instrument apparatus;
FIG. 15 is a cutout perspective view of disposable shutoff valve apparatus used in conjunction with a surgical suction device;
FIG. 16 is a longitudinal cross-sectional view of the disposable shutoff valve apparatus;
FIG. 17 is an exploded side elevational view of the disposable shutoff valve apparatus shown in FIG. 15;
FIG. 18 is a partial perspective view of a suction hose clamping mechanism of the disposable shutoff valve apparatus;
FIG. 19 is a partial perspective view of another suction hose clamping mechanism of the disposable shutoff valve apparatus;
FIG. 20 is a perspective view of another disposable shutoff valve apparatus used in conjunction with a surgical suction device;
FIG. 21 is a longitudinal cross-sectional view of the disposable shutoff valve apparatus shown in FIG. 20;
FIG. 22 is an exploded side elevational view of the disposable shutoff valve apparatus shown in FIG. 20;
FIG. 23 is a perspective view of the flexible sleeve retained thereto by two retaining members of the disposable shutoff valve apparatus;
FIG. 24 is a perspective view of an alternative flexible sleeve used with the disposable shutoff valve apparatus shown in FIG. 20;
FIG. 25 is a perspective view of another embodiment of the disposable shutoff valve apparatus.
FIG. 26 is a perspective view of the improved retaining member to retain the first and second ends of the elongated flexible sleeve inserted into the suction chamber, the suction chamber made of transparent material so that the retaining members and flexible sleeve are visible;
FIG. 27 is a front view of the improved second retaining member and its diaphragm used to retain the second open end of the flexible member in the suction chamber;
FIG. 28 is a perspective view of only the second retaining member and retaining the flexible sleeve within the suction chamber, the retaining member visible because the suction chamber is made of transparent material;
FIG. 29 is a closeup perspective view of the front end of the flexible sleeve being retained by an improved retaining member and further retained by a ball placed at the front end of the flexible sleeve which is retained in a separate chamber in the suction chamber;
FIG. 30 is a front elevational view of an improved retaining mechanism for retaining the front end of the flexible sleeve;
FIG. 31 is a perspective view of an improved clamping mechanism to retain the suction hose;
FIG. 32 is an open perspective view of an embodiment of the present invention as formed by an improved manufacturing method;
FIG. 33 is a closed view of an embodiment of the present invention as manufactured by an improved method;
FIG. 34 is a side elevational view of an embodiment of the present invention with an improved flexible extension added thereto; and
FIG. 35 is a perspective view of an improvement in the longitudinal channel to enable debris to be removed from the cautery.

### DETAILED DESCRIPTION OF THE PREFERRED EMBODIMENTS

Although specific embodiments of the present invention will now be described with reference to the drawings, it should be understood that such embodiments are by way of example only and merely illustrative of but a small number of the many possible specific embodiments which can represent applications of the principles of the present invention. Various changes and modifications obvious to one skilled in the art to which the present invention pertains are deemed to be within the spirit, scope and contemplation of the present invention as further defined in the appended claims.

In the following description of the preferred embodiment, reference is made to the accompanying drawings which form a part hereof, and in which is shown by way of illustration a specific embodiment in which the present invention may be practised. It is to be understood that other embodiments may be utilized and structural changes may be made without departing from the scope of the present invention.

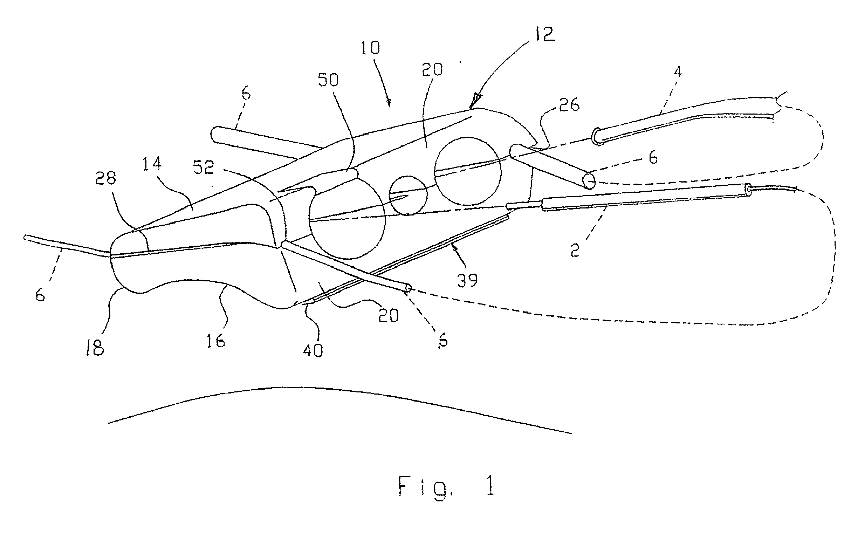

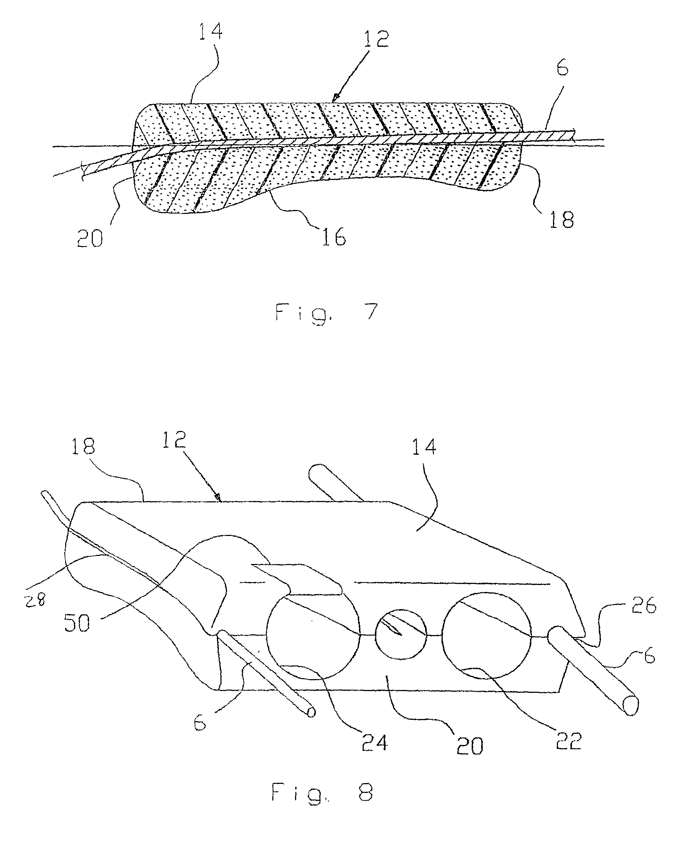

Referring to FIGs. 1 and 8, there is shown a disposable surgical instrument holder and cleaner apparatus referred to generally by the reference numeral 10. The holder and cleaner apparatus 10 can securely retain surgical instruments 2 and 4 when not in use during surgery. The apparatus 10 comprises a generally rectangular shaped holder body 12 which has a top surface 14, a contoured bottom surface 16, a thin rear end 18, a thick common end 20, one or more longitudinal elongated cylinders 22 and 24 disposed adjacent one another, and one or more elongated open slotted channels 26 and 28 respectively located adjacent to the elongated cylinders 22 and 24.

A surgeon workstation is provided which can securely hold a plurality of surgical instruments, such as a suction device 4 and its suction hose 6, a cautery 2 and its electrical cord 8 or other surgical instruments (e.g., a diathermy pencil and laparoscopy instruments) used in surgery that are needed to be in close proximity to the surgeon, some of which the surgical instruments are attached to a fiber-optic cable, hose or electrical cord (hereafter referred to as "extensions").

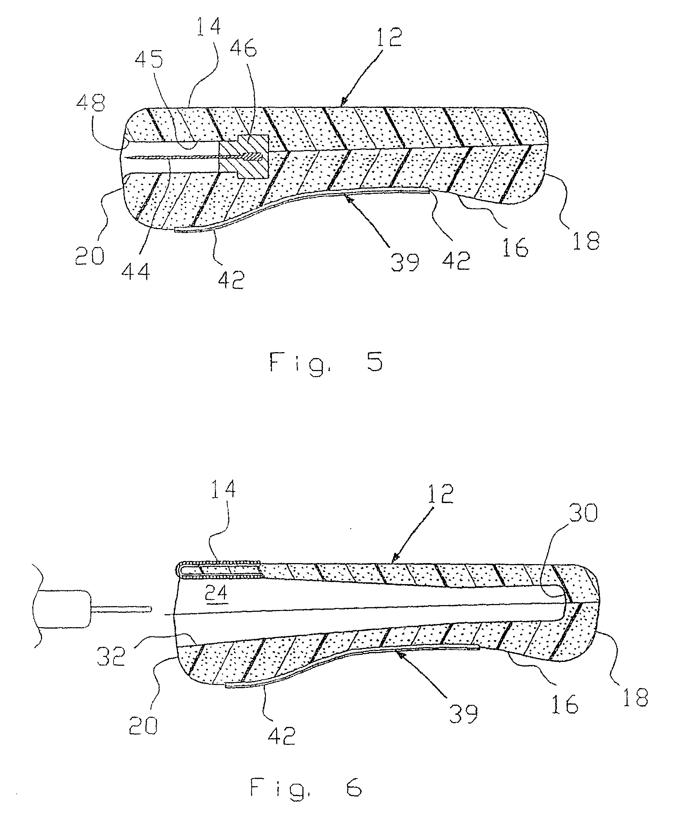

Referring to FIGs. 1, 4 and 6, each elongated cylinder has a closed end 30 and a conical open end 32 located at the thick common end 20 of the holder body 12 for ease of insertion of the surgical instruments and sized to hold the surgical instruments when not in use during surgery, but allow easy removal and replacement of the surgical instruments, such as a suction device 4, a diathermy pencil, laparoscopic instruments or the like. The elongated suction cylinder 22 provides means for easily and automatically shutting off the suction nozzle 5 of the suction device 4 when not in use and eliminating the objectionable noise from the suction nozzle 5 of the suction device 4. The shut-off means has a sealed airtight chamber 34 with a passageway 36 that decreases in diameter 38 for accommodating a plurality of different sized suction nozzles, where the sealed airtight chamber 34 stops the suction action and muffles the noise from the suction nozzle 5 of the suction device 4. The passageway 36 of the elongated cylinder 22 is contoured so as to accept and seal suction nozzles of different sizes and shapes. The passageway 36 of the cylinder 22 has a smaller diameter than the diameter of the suction nozzle 5 for insertion of the suction nozzle, such that the suction nozzle can be securely held and at the same time be sealed without the need for a diaphragm. The passageway 36 is progressively narrower so that a narrow suction nozzle will find anchorage at a deeper level of the cylinder 22 and a wider suction nozzle will meet resistance and anchorage at a lesser depth of the cylinder 22. The elongated cautery cylinder 24 securely holds the cautery or the like in place when not in use during surgery, but allows easy removal and replacement of the cautery. The two separate elongated cylinders 22 and 24 decrease the possible airborne contaminations of the nozzle tip. The longitudinal elongated cylinder 24 can also be made by having the cylinder 24 extend through the entire body 12 with openings at both ends to accommodate a longer surgical instrument thereto.

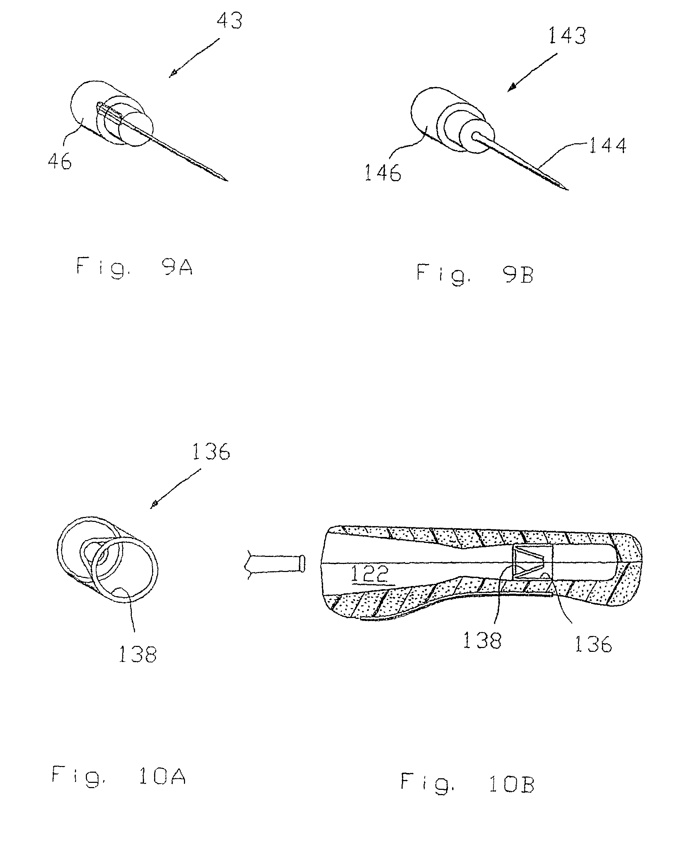

Referring to FIGs. 10A and 10B, there is shown an alternative embodiment of the suction cylinder 122 of the holder and cleaner apparatus.

Alternatively, a diaphragm or insert 136 can be inserted along the length of the suction cylinder 122, where the diaphragm 136 has an opening 138 for the passage of the suction nozzle of a suction device. The hole 138 has a progressively smaller diameter to accommodate different sized suction nozzles.

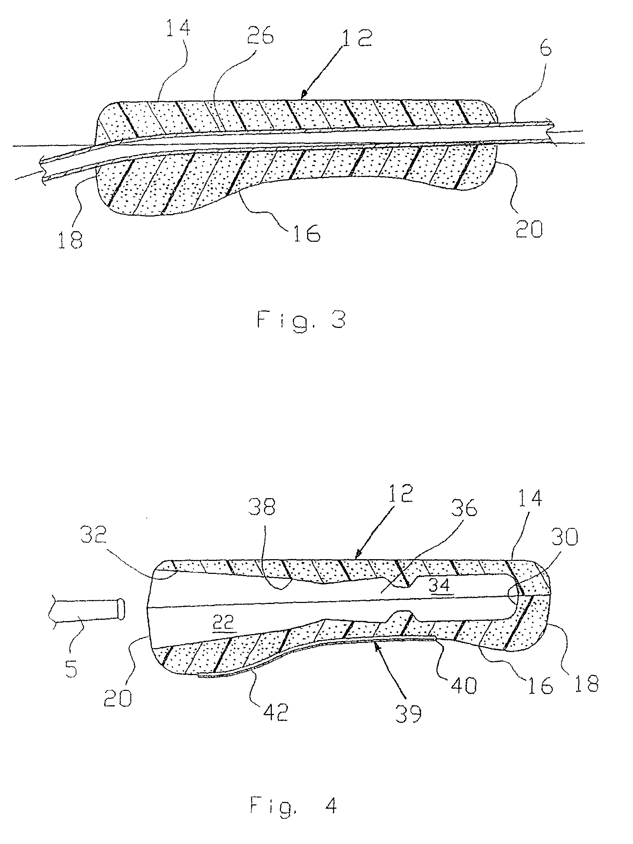

Referring to FIGs. 3, 7 and 8, the two elongated open slotted channels 26 and 28 are respectively located adjacent to the elongated cylinders 22 and 24, and extend lengthwise of the holder body 12 for respectively anchoring and directing the extensions 6 of the suction device 4 and cautery 2 of the surgical instruments. Each slotted channel has an angular opening at the thick common end 20 of the holder body 12 for anchoring and directing the extension 6 downwardly away from the surgeon during surgery. The angular opening has an angle of approximately 5 to 15 degrees (see FIGs. 3 and 7). This angular opening is made possible by the thick common end 20 of the holder body 12. The extensions 6 are held in a predetermined place away from a surgical area but are movable in response to manipulation of the surgical instruments. The channels 26 and 28 are angled to force direction on the emerging extensions. Each slotted channel has a beveled slit or flange 60 which leads to the channel and accommodates the extension of different diameters. The flange 60 prevents the extension from popping out of the channel. The slotted channels 26 and 28 further provide safe and speedy hose or cable detachment and re-attachment to the holder body 12 should the suction device or cautery instruments fall on the floor.

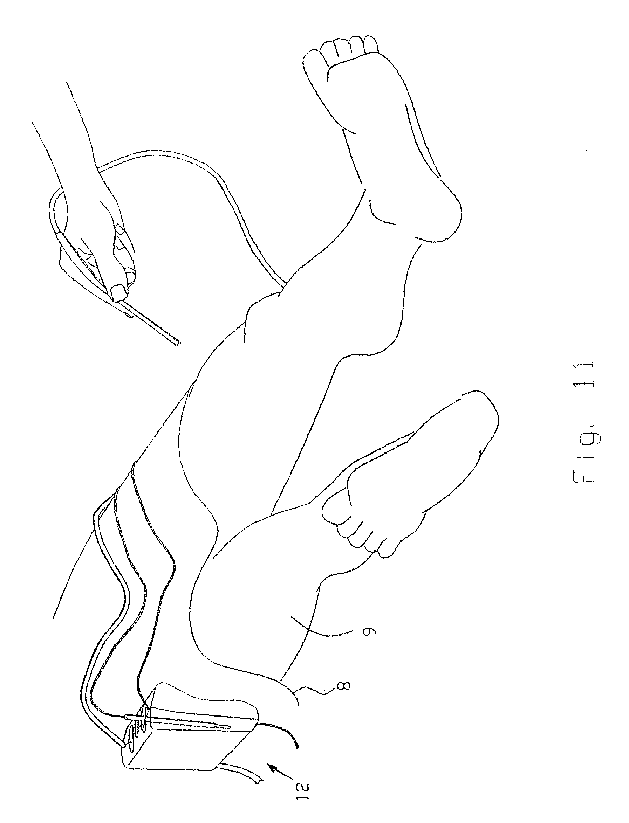

Referring to FIGs. 1, 3, 4, 5 and 6, there is shown a pressure sensitive adhesive means 39 which includes an adhesive surface 40 covered by a removable cover 42. The pressure sensitive adhesive means 39 is affixed to the contoured bottom surface 16 of the holder body 12 adjacent to the thick common end 20. When the removable cover 42 is removed from the adhesive surface 40, the holder body 12 can be secured to a surgical drape 8 around a patient 9 (see FIG. 11). The pressure sensitive adhesive means 39 provides for secure and quick attachment to the sterile drape 8 at a site convenient to the surgeon. The apparatus 10 may be attached to the patient directly, to the drape around the patient, incorporated into the drape or even attached to a convenient stand or table adjacent to the operating room table.

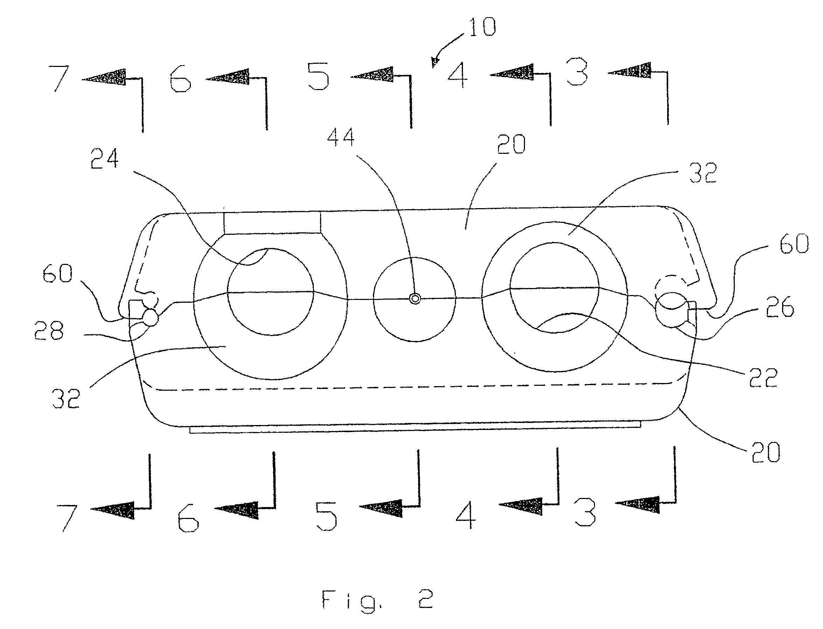

Referring to FIGs. 2, 5 and 9A, there is shown a cleaning means 43 which includes a cleaning spike or pin 44 attached to a butt insert 46. The butt insert 46 can be press-fitted or integrally molded within a narrow chamber 45 of the holder body 12. The cleaning spike 44 is used for dislodging clogged soft tissue or bone fragments from the suction nozzle. The narrow chamber 45 is located parallel and between the two elongated cylinders 22 and 24. The chamber 45 has a conical opening 48 at the common end 20 of the holder body 12 for ease of insertion of the suction nozzle of the suction device 4, such that the suction nozzle of the suction device can be inserted into the cleaning spike 44 for prying and cleaning loose obstructions in the suction nozzle of the suction device 4. The cleaning spike 44 can be made of metal material as shown in FIG. 9A. The components of the cleaning means 143 can be integrally molded into a one-piece plastic component which includes a cleaning spike 144 and a butt insert 146 as shown in FIG. 9B.

Referring to FIGs. 1 and 8, there is shown a small abrasive pad 50 which is affixed on the top surface 14 of the holder body 12 of the apparatus 10 for cleaning a tip of the cautery 2 and scraping char off the tip of the cautery. This abrasive pad 50 provides more efficient means for removing the char from the active cautery electrode tip. The abrasive pad 50 has two abrasive surfaces 52 at an angle to each other for cleaning the tip of the cautery.

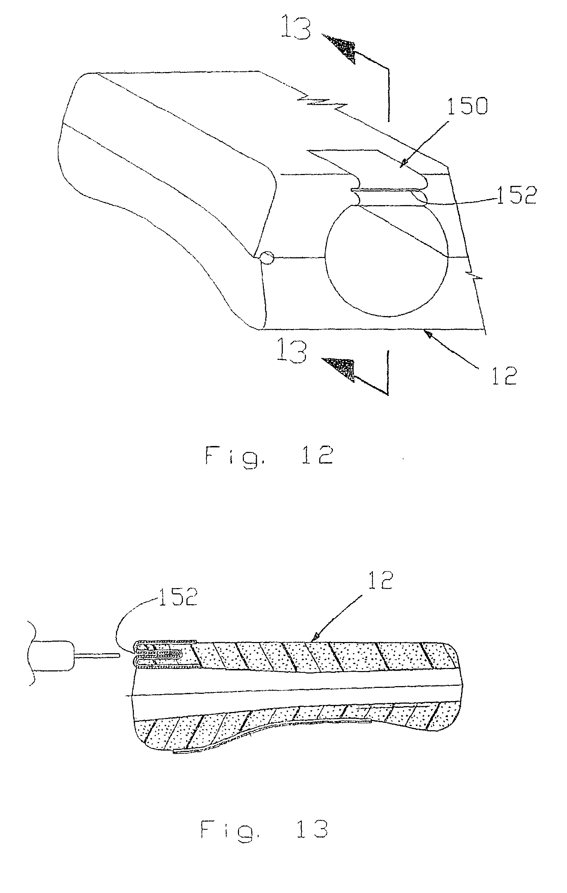

Referring to FIGs. 12 and 13, there is show an alternative abrasive pad 150 which is integrally mounted to the holder body 12 and a slit 152 which is formed to allow the insertion of the tip of cautery to be cleaned in one motion.

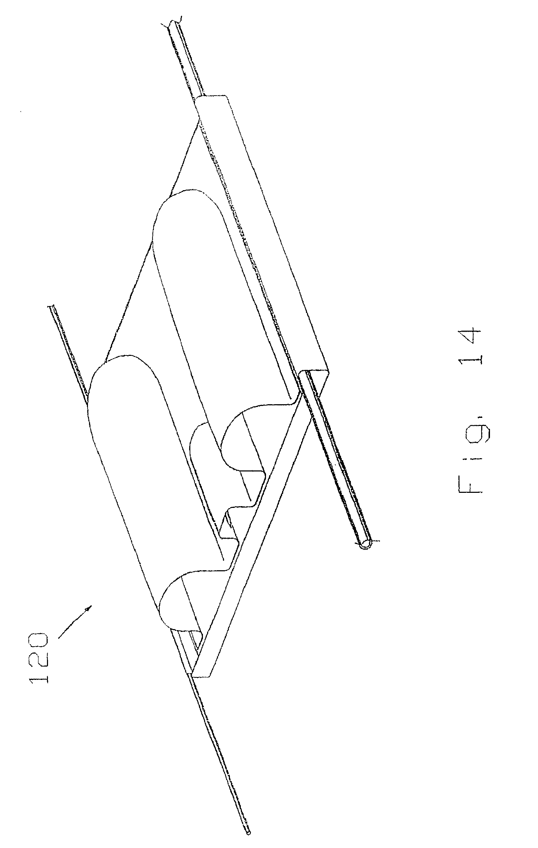

Referring to FIG. 14, there is shown an alternative method of constructing the disposable surgical instrument holder and cleaner apparatus 110. The method is vacuum formed from vinyl urethane.

Thus, a secure, sterile, light-weight, flame-retardant, non-conducting, non-toxic apparatus is provided, which converts the generally irregular, convex, unusable, unstable surface over a draped surgical patient into a usable, stable, working space for the surgeon. The entire apparatus 10 can be discarded at the end of the operation.

The apparatus 10 may be constructed from a generally block of flexible semi-rigid foam material. The foam walls of the apparatus 10 provide physical, electrical and thermal insulation from the patient. It is emphasized that while the flexible foam material is preferred, it is also possible to use other materials, such as plastic foam material, urethane material, cross-linked polyethylene foam material or any other suitable material. The apparatus 10 is being conveniently package sterile in a strippable sterile package.

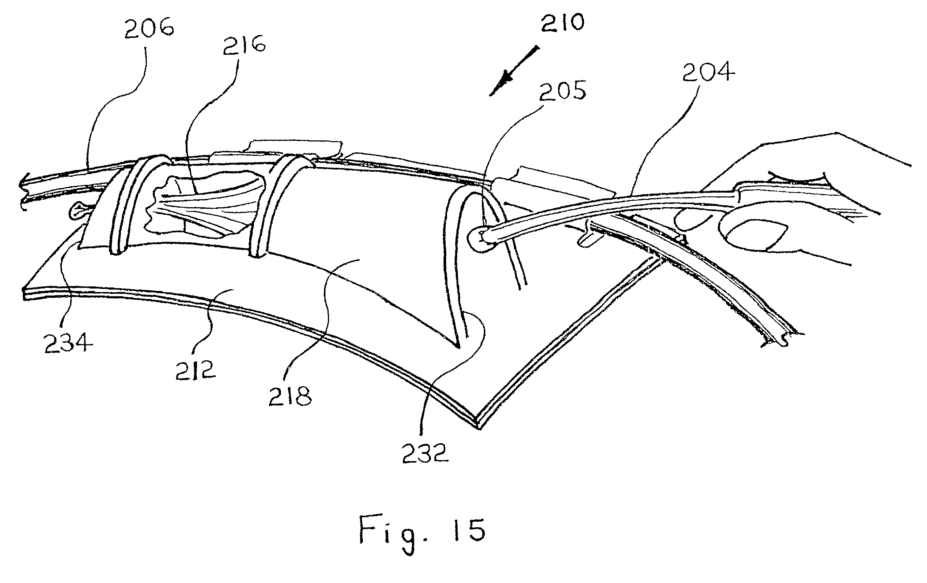

Referring to FIG. 15, there is shown disposable shutoff valve apparatus 210 which is used in conjunction with a surgical suction device 204 for stopping the suction action and muffling the noise from the suction device 204 during surgery. The suction device 204 includes a suction nozzle 205 and a suction hose 206.

Referring to FIGs. 15, 16 and 17, the apparatus 210 includes an upper portion 212, a base portion 214 and an elongated flexible sleeve or member 216. The base portion 214 forms a generally rectangular shaped body which has a contoured support surface 220. The contoured support surface 220 has an upper side 222 and a lower side 224.

The lower side 224 is provided with pressure sensitive adhesive material 260 thereon and covered by a peel-off cover 264 which can be peeled off so that the adhesive material 260 can be securely attached to a surgical drape around a patient during surgery or other stationary object.

Referring to FIGs. 15, 16, 17, and 18, the upper portion 212 forms a generally rectangular shaped body with a bottom side 217 and a longitudinal U-shaped upside down channel 218 extending upwardly thereto. The upper portion 212 further has a hose clamping mechanism 221 located adjacent to the U-shaped upside down channel 218 for securing and retaining the suction hose 206 of the suction device 204 to the apparatus 210.

What is unique about the hose clamping mechanism 221 is that it only requires a finger of a user to push up a tab member 223 such that the suction hose 206 slides within housing of the hose clamping mechanism 221. A stiffer means 290 is integrally formed with the tab member 223 for firming the tab member 223 when it is pushed upwardly to clamp the hose 206 of the suction device 204 to the apparatus 210.

The apparatus 210 is formed by affixing the bottom side 217 of the upper portion 212 to the upper side 222 of the base portion 214 by conventional means. Once the two portions 212 and 214 are attached together, a longitudinal suction chamber 230 is formed. The suction chamber 230 has an open end 232 and a closed end 234. The suction chamber 230 further has two spaced apart transverse grooves 240 and 242.

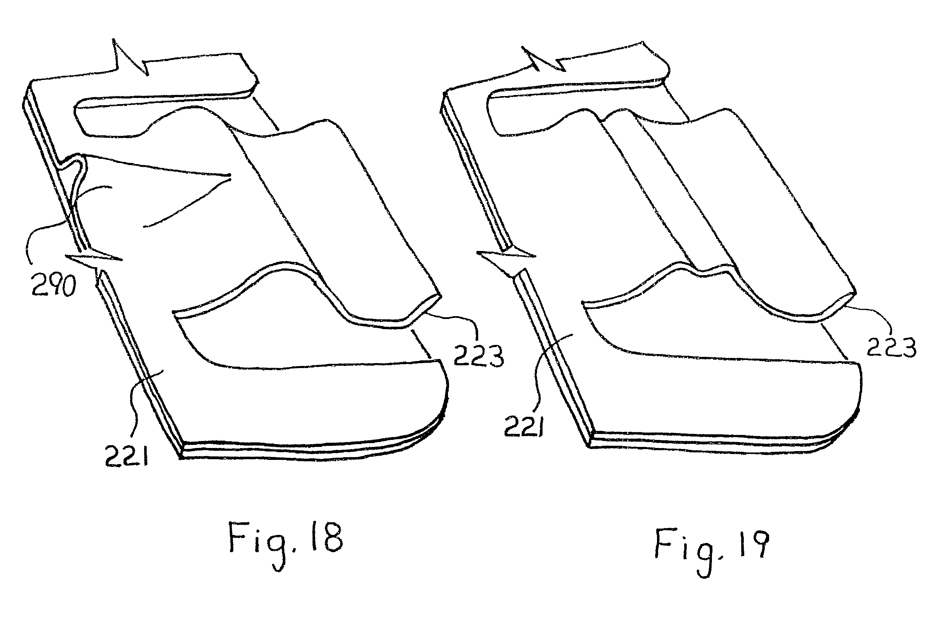

Referring to FIG. 19, there is shown another suction hose clamping mechanism 221 which is similar to the clamping mechanism shown in Figure 18.

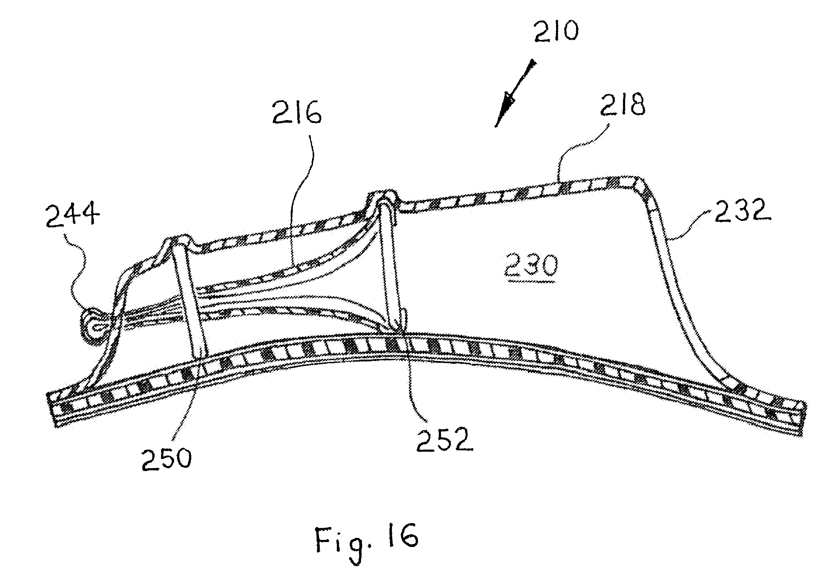

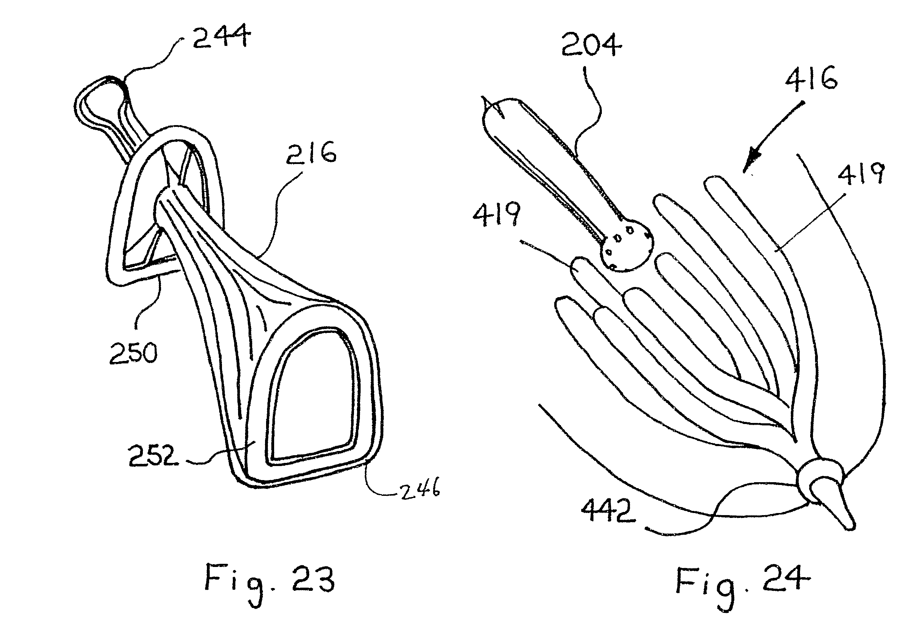

Referring to FIGs. 16 and 23, there is shown the elongated flexible sleeve 216 which has a first end 244 and a second end 246, where the flexible sleeve 216 is installed and disposed at an approximate midsection of the suction chamber 230 and secured thereto by two retaining members 250 and 252. The retaining members 250 and 252 conform to the spaced apart transverse grooves 240 and 242 formed on the interior wall of the suction chamber 230. The first end 244 of the flexible sleeve 216 is inserted into and held by the lower retaining member 250 while the second end 246 of the flexible sleeve 216 encompasses the upper retaining member 252 and press-fitted and held by the transverse groove 242.

Referring to FIG. 24, there is shown another flexible member 416 which is shown in FIG. 23 as reference number 216. In Fig. 24, the flexible member 416 is made out of Mylar? material and has a plurality of straps or strings 419. This flexible member 416 functions the same as the preceding embodiment by having the suction device 204 engage the plurality of straps 419 toward itself, and thereby stops the suction action of the suction device and muffles the noise from the suction nozzle of the suction device for automatically shutting off the suction nozzle of the suction device. The flexible member 416 has a knot front end 442 that is inserted through the aperture located on the suction chamber 230 and affixed within the suction chamber.

Referring to FIGs. 15 and 16, the apparatus 210 is attached on the surgical drape around the patient by the pressure sensitive adhesive means 260. The suction nozzle 205 of the suction device 204 can be inserted into and through the open end 232 of the longitudinal suction chamber 230 and passes through the upper retaining member 252 such that the suction nozzle 205 pulls the flexible sleeve 216 toward itself, and thereby the flexible sleeve 216 stops the suction action of the suction nozzle 205 and muffles the noise from the suction nozzle 205 of the suction device 204 and further simultaneously grips the suction device within the suction chamber.

The entire apparatus 210 is made out of polyethylene terephthalate (PET) material while the flexible sleeve 216 is made out of latex material. It is emphasized that while the materials mentioned above are preferred, it is also possible to use other suitable materials known in the art. For example, the flexible sleeve 216 can be made out of Mylar material. The apparatus 210 is being conveniently package sterile in a strippable sterile package.

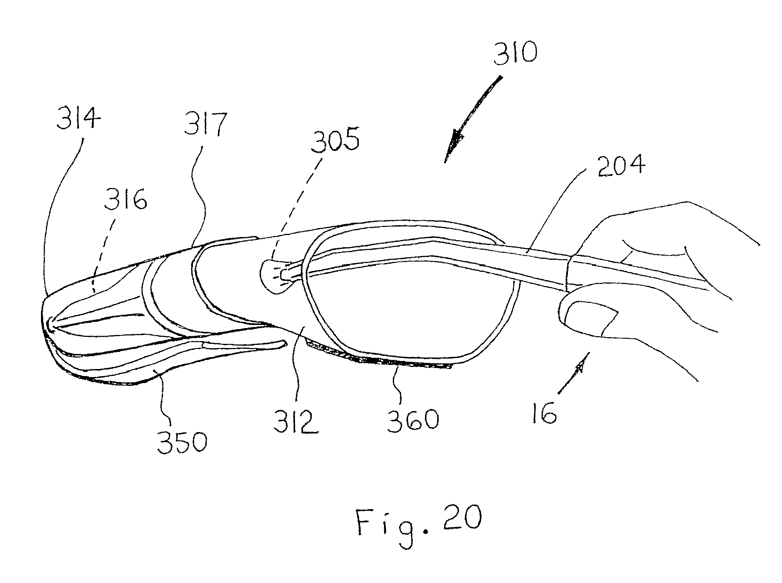

Referring to FIG. 20, there is shown a further disposable shutoff valve apparatus 310 which is used in conjunction with a surgical suction device 204 having a suction nozzle 205. The shutoff valve apparatus 310 is utilized for stopping the suction action and muffling the noise from the suction device 204. The apparatus 310 comprises a hollow upper or first portion 312, a hollow lower or second portion 314 and a generally flexible sleeve 316. When the lower portion 314 is attached to the upper portion 312, they will form a generally longitudinal elliptical-shaped body 317 which encloses the flexible sleeve 316.

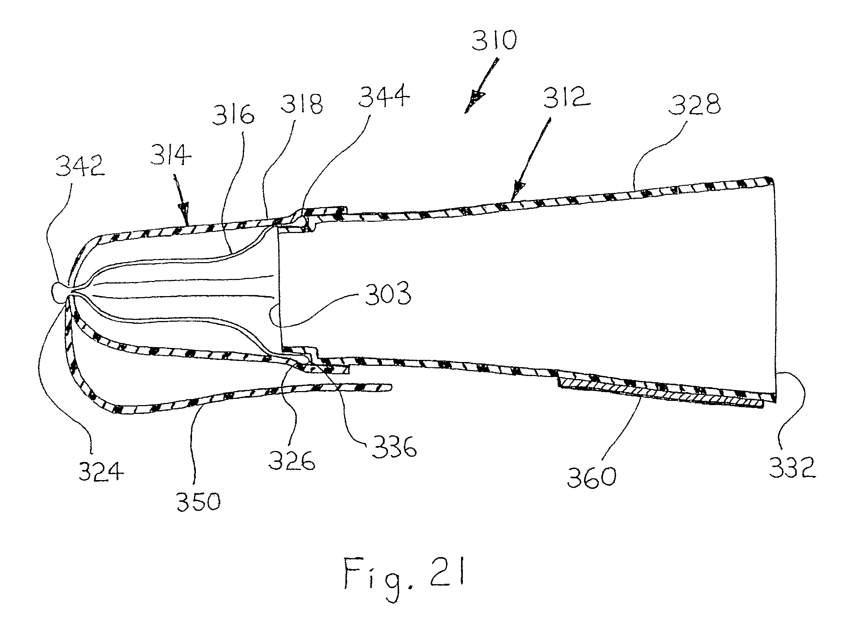

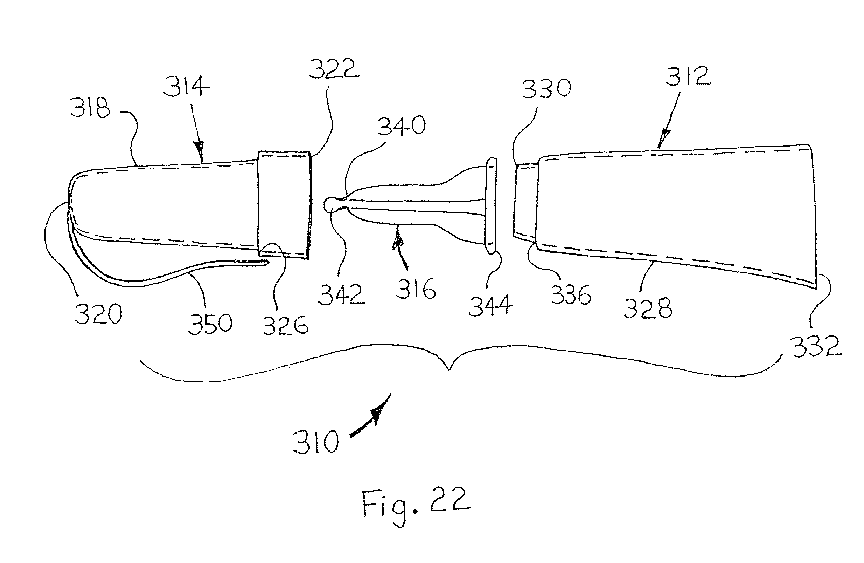

Referring to FIGs. 21 and 22, the hollow lower portion 314 includes a circumferential sidewall 318, a closed front end 320 and an open rear end 322. The front end 320 has a small central aperture 324 that extends therethrough. The open rear end 322 has an interior ledge 326 that has a diameter larger than a diameter of the circumferential sidewall 318 of the lower portion 314. The lower portion 314 further includes a hook or fastener means 350 which is integrally formed with the circumferential sidewall 318 and located adjacent to the closed front end 320. The hook means 350 is used for fastening the shutoff valve apparatus 310 to a stationary object such as to a patient directly, to the drape around the patient, incorporated into the drape or even attached to a convenient stand or table adjacent to the operating room table.

The hollow upper portion 312 includes a circumferential sidewall 328, an open front end 330 and an open rear end 332 which extends through to the front end 330. The front end 330 has an exterior ledge 336 that has a diameter smaller than a diameter of the circumferential sidewall 328. A pressure sensitive adhesive means 360 is affixed to the circumferential sidewall 328 adjacent to the rear end 332 of the upper portion 312 and has an adhesive surface 362 covered by a removable cover 364 for securing the longitudinal body 317 of the disposable shutoff valve apparatus 310 to a stationary surface.

The flexible sleeve 316 has a closed front end 340 with a retaining tip 342 and a ring-like shaped rear end 344 and sized to fit over the front end 330 of the upper portion 312. The flexible sleeve 316 is installed within the hollow lower portion 314 such that the retaining tip 342 is inserted through the small central aperture 324 and retained thereto while the ring-like shaped rear end 344 of the flexible sleeve 316 encompasses the front end 330 of the hollow upper portion 312 and situated on the ledge 336. The upper and lower portions 312 and 314 are press-fitted together such that the rear end 322 of the lower portion 314 is locked with the front end 330 of the upper portion 312, thereby securely trapping the ring-like shaped rear end 344 of the flexible sleeve 316 between the upper and lower portions 312 and 314.

The suction device 204 can be inserted into and through the upper and lower portions 312 and 314 of the apparatus 310 such that the flexible sleeve 316 pull towards the suction nozzle 205, and thereby stops the suction action of the suction device 204 and muffles the noise from the suction nozzle 205 of the suction device 204 for automatically shutting off the suction nozzle 205 of the suction device 204.

The upper portion 312 and lower portion 314 of the shutoff valve apparatus 310 may be formed from plastic materials while the flexible sleeve 316 is made out of latex material. It is emphasized that while the materials mentioned above are preferred, it is also possible to use other materials, such as urethane material for the upper portion 312 and lower portion 314 of the shutoff valve apparatus, and Mylar material for the flexible sleeve 316. The apparatus 310 is being conveniently package sterile in a strippable sterile package.

Referring to FIG. 24, there is shown the flexible member 416 which is similar to the flexible sleeve 316 shown in FIGs. 20, 21 and 22. In this embodiment, the flexible member 416 is made out of Mylar material and has a plurality of straps or strings 419. This flexible sleeve 416 functions the same as the preceding embodiment by having the suction device 204 engage the plurality of straps 419 toward itself, and thereby stops the suction action of the suction device and muffles the noise from the suction nozzle of the suction device for automatically shutting off the suction nozzle of the suction device. The flexible sleeve 416 has a knot front end 442 that is inserted through the aperture located on the lower portion of the apparatus and retained thereto (see Figure 21).

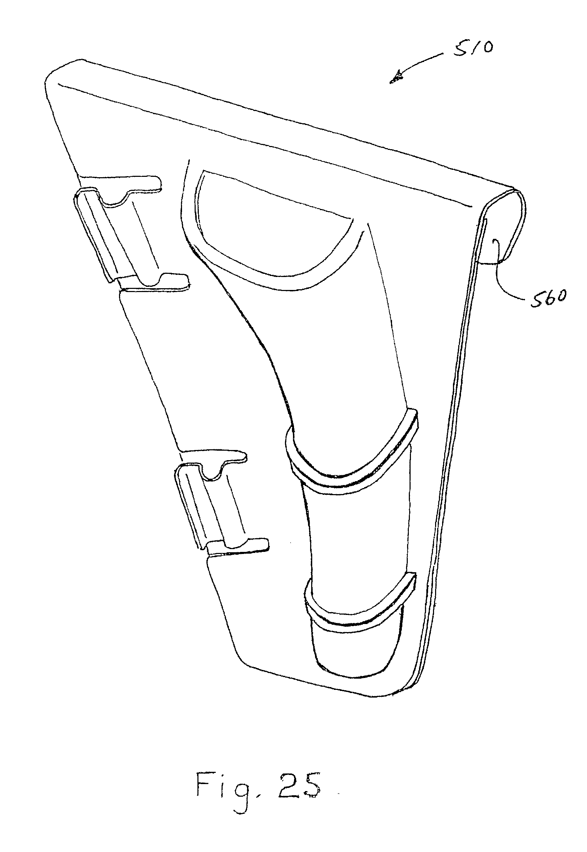

Referring to FIG. 25, there is shown another disposable shutoff valve apparatus 510 which is similar to that shown in FIG. 15. The only difference between the two embodiments is that Figure 25 shows a hooking mechanism 560 for attaching to a stationary object during surgery and the description thereof will not be repeated.

Referring to FIGs. 15 and 16, the disposable shutoff valve apparatus 210 is attached on the surgical drape around the patient by pressure sensitive means. The suction nozzle 205 of the suction device 204 is inserted into and through the open end 232 of the longitudinal suction chamber 230 and passes through the upper retaining member 252 into the flexible sleeve 216 where the suction nozzle pulls the flexible sleeve toward itself, and thereby the flexible sleeve stops the suction action of the suction nozzle and muffles the noise from the suction nozzle and further simultaneously grips the suction nozzle and device within the suction chamber. However, the suction nozzle will not pull the flexible sleeve 216 towards itself if it is not in contact with, and surrounded by the sleeve 216. It will readily be appreciated from FIG. 16, that in this embodiment the nozzle 205 will only be sufficiently surrounded by the sleeve 216 when it approaches the lower retaining member (250) where the interior space of the sleeve 216 becomes narrow.

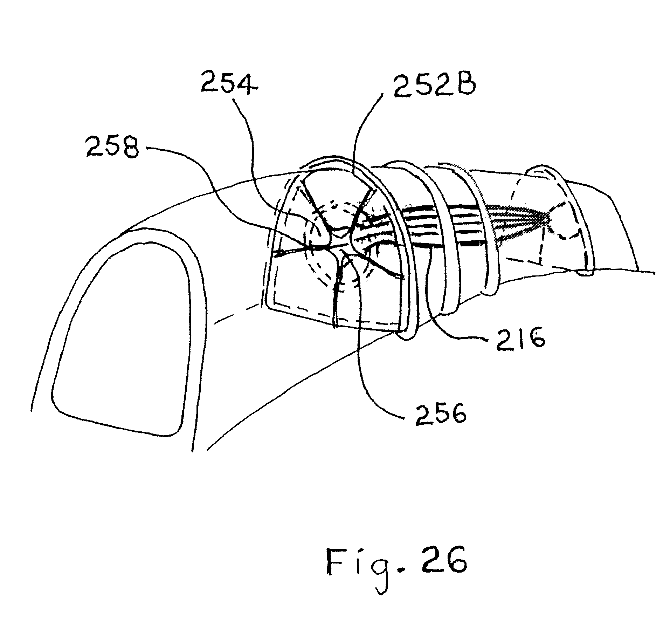

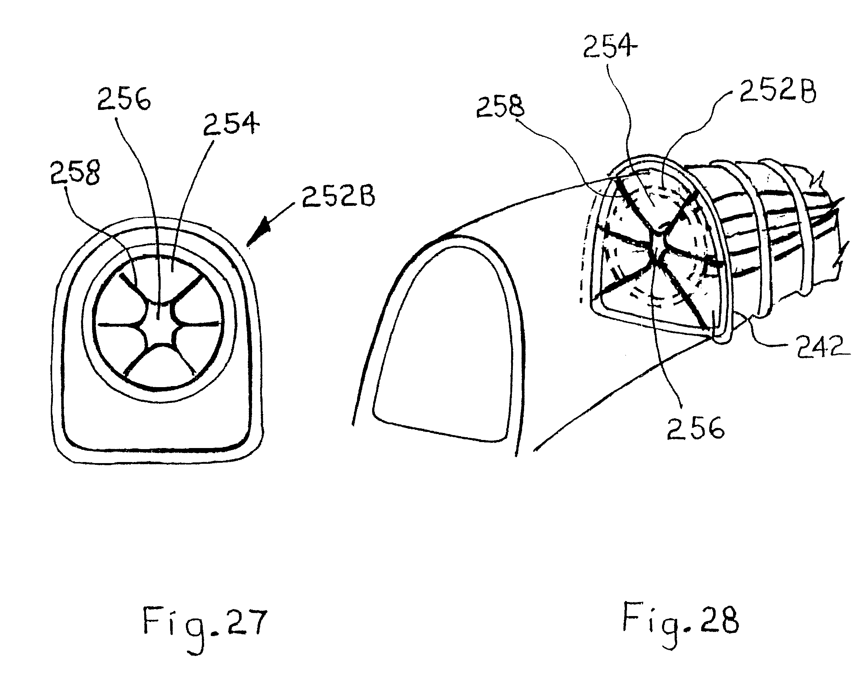

Referring to Figures 26, 27 and 28, the problem is solved by providing means for gathering the sleeve 216 into a narrowed segment somewhere near its open end. In one such embodiment, the second retaining member 252B is converted into a diaphragm 254 and the second open end of the sleeve 216 is caused to pass through the center opening 256 of the diaphragm 254 and then the rim of the upper open end of the sleeve 216 is folded over and down over the retaining member 252B before the retaining member 252B is set into the transverse groove 242 formed on the interior wall of the suction chamber 230. The diaphragm 254 has a plurality of radial slits 258 which surround the opening 256 to allow easy passage of the nozzle while keeping the sleeve 216 gathered into a narrow passage. The retaining member 252B and its diaphragm 254 having a multiplicity of slits 258 surrounding the central opening 256 and has a dual function as a diaphragm and as a second retaining member.

The diaphragm 254 gathers the sleeve 216 into a narrow passage at the second retaining member, and the nozzle 205 passes into this narrow passage as it passes the second retaining member 252B and moves down into the sleeve 216. The nozzle 205 is thereby closely surrounded by the sleeve 216 as it passes through the second retaining member 252B, Negative pressure generated within the sleeve 216 by the suction device 204 further pulls the sleeve towards the nozzle 205, the sleeve 216 is forced by negative pressure onto the surface of the nozzle 205 and the surface of the suction device 204, the sleeve thereby grips and retains the nozzle 205 and suction device 204, seals off the apertures in the nozzle 205, and thereby acts as a shutoff valve, stopping the suction action of the suction nozzle, and muffling or shutting off the hissing sound of the nozzle 205. In another embodiment a rubber band is placed around the sleeve 216 somewhere near its upper open end, thus providing a narrowed constriction through which the suction nozzle 205 must pass.

In addition to the improvement in the rear end of the retaining member having the multiplicity of slits in a diaphragm to better retain the rear end of the flexible sleeve 216, the present inventor has also conceived of improved anchorage for the first end of the flexible sleeve to better retain the flexible sleeve within the suction chamber 230.

Referring to FIGs. 16 and 23, there is shown the elongated flexible sleeve 216 which has a first end 244 and a second end 246, where the flexible sleeve 216 is installed and disposed at an approximate midsection of the suction chamber 230 and secured thereto by two retaining members 250 and 252 or 252B. The retaining members 250 and 252 (or 252B) conform to the spaced apart transverse grooves 240 and 242 formed on the interior wall of the suction chamber 230. The first end 244 of the flexible sleeve 216 is inserted into and held by the first retaining member 250 while the second end 246 of the flexible sleeve 216 encompasses the second retaining member 252 and is press-fittedly held between the transverse groove 242 and the retaining member 252 (or retained by improved retaining member 252B as previously described).

In this embodiment, the first end 244 of the flexible sleeve 216 is secured to the retaining member 250 by passing the first end 244 through slits in the retaining member 250. This arrangement is difficult to assemble, can result in tearing of the sleeve during assembly, and provides inadequate retention for the first end during the functioning of the device.

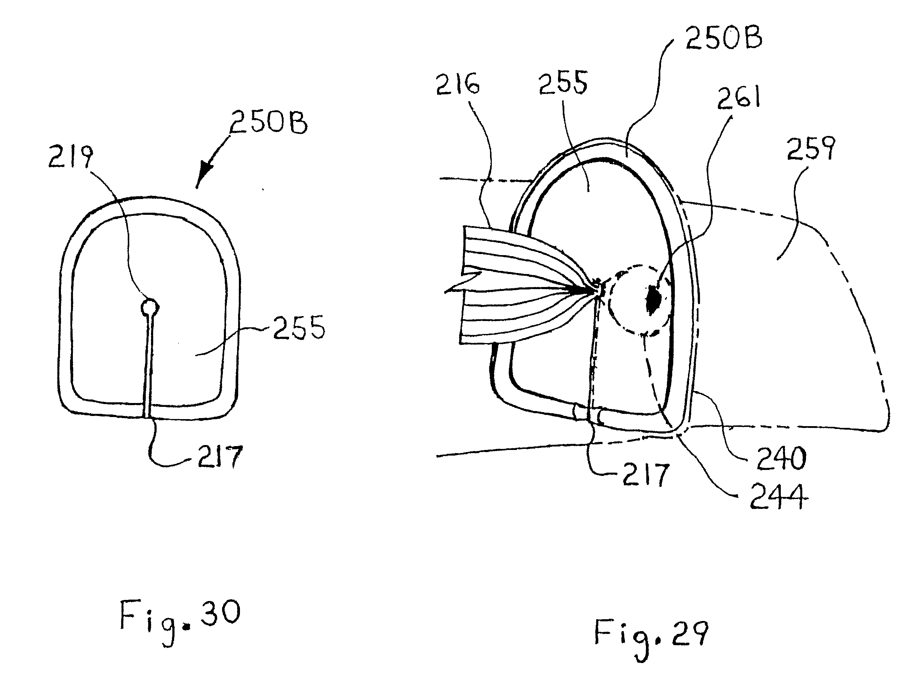

Referring to FIGs. 29 and 30, the mechanism for retaining the first end of the sleeve is improved by providing the retaining member 250B with a single slit 217 that breaches the surface 255 of the retaining member 250B and terminates in an opening 219. When the retaining member 250B is placed into the transverse groove 240 formed on the interior wall of the suction chamber 230, it forms a separate chamber 259 at the closed end of the suction chamber 230. During assembly of the device, the retaining member 250B with its slit 217 is set into its transverse groove 240. An interior retaining member which, by way of example, can be a spherical plastic ball 261 that has a larger diameter than the width of the slit 217 and the opening 219 is placed into the sleeve 216 and allowed to drop into the first end 244 of the sleeve. The first end of the sleeve 244 with its contained spherical plastic ball is now placed in the separate chamber 259, and the sleeve adjacent to the spherical plastic ball is bunched up into the single slit 217 in the retaining member 250B. When the upper portion 212 and the base portion 214 of the suction chamber are affixed to each other, the separate chamber 259 is sealed off and the spherical plastic ball 261, which is trapped in the sealed chamber 259, efficiently retains the first end 244 of the sleeve 216.

Referring to FIGs 15, 18 and 19 the upper portion 212 has a hose clamping mechanism 221 located adjacent to the U-shaped upside down channel 218 for securing and retaining the suction hose 206 of the suction device 204 to the apparatus 210. The hose clamping mechanism 221 only requires a finger of a user to push up a tab member 223 such that the suction hose 206 slides within housing of the hose clamping mechanism 221.When a thick plastic sheet is used for the construction the apparatus 210 the tab as shown in FIG 19 of has sufficient rigidity and functions well. When a thinner plastic is used it has insufficient springiness to clamp the hose securely and a stiffer means 290 is integrally formed with the tab member 223 for firming the tab member 223 when it is pushed upwardly to clamp the hose 206 of the suction device 204 to the apparatus210.

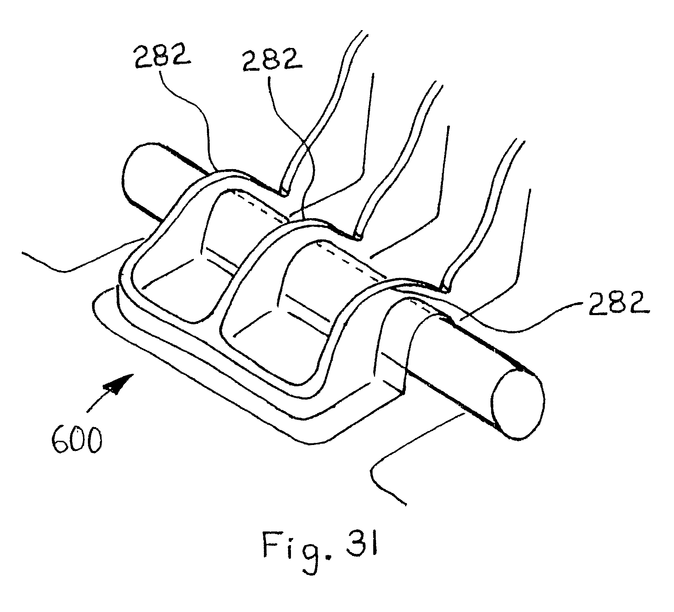

For cost containment it is desirable to use the thinnest possible plastic sheeting for the construction of the apparatus 210. If the apparatus 210 is made without the clamping mechanism 221 plastic sheeting as thin as twenty five thousandths of an inch is workable. However at that thickness the hose clamping mechanism is too thin to grip the suction hose 206 even with the stiffer means 290 incorporated. Adding one or more ribs to the clamping mechanism running the width of the hose clamping mechanism will stiffen the mechanism but render it too stiff to function well because the tab will not easily lift. Referring to Figure 31, if each rib 282 is interrupted as it passes over the hose-gripping channel 218, then the tab will lift off easily. If the interruptions are in a single line on the circumference of the suction tube-gripping channel, the tab lifts too easily and does not function well. If the interruptions are staggered as shown in Figure 31, the plastic is intrinsically distorted as the tab is lifted and this distortion provides sufficient resistance to render the hose clamping mechanism functional using a very thin plastic for the manufacture of the apparatus.

Referring to FIGs. 15, 16 and 17, the apparatus 210 includes an upper portion 212, and a base portion 214. Referring to FIGs. 15, 16, 17, and 18 the upper portion 212 forms a generally rectangular shaped body with a bottom side 217 and a longitudinal U-shaped upside down channel 218 extending upwardly thereto. The apparatus 210 is formed by affixing the bottom side 217 of the upper portion 212 to the upper side 222 of the base portion 214 by conventional means. Once the two portions 212 and 214 are attached together, a longitudinal suction chamber 230 is formed. The suction chamber 230 has an open end 232 and a closed end 234.The means for affixing the bottom side 217 of the upper portion 212 to the upper side 222 of the base portion 214 is not specified.

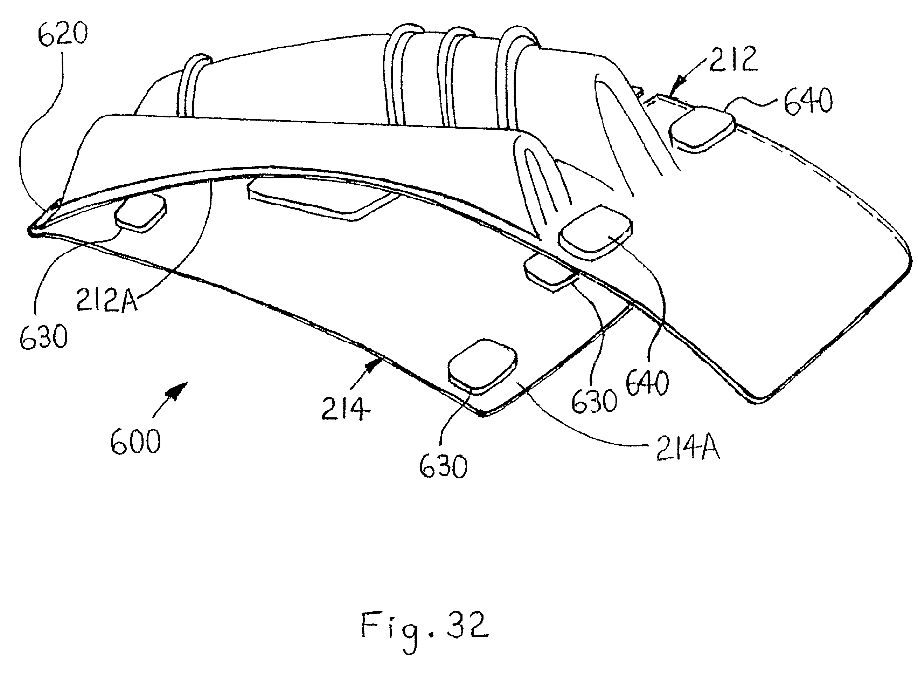

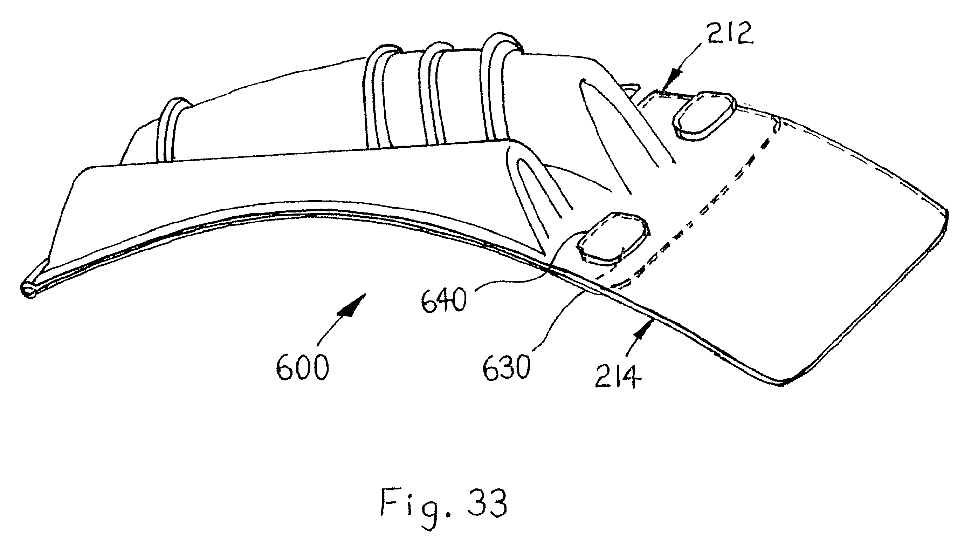

Referring to FIGs. 32 and 33, the means for manufacture of the apparatus 210 and affixing upper portion 212 to the base portion 214 of the apparatus are as follows: The upper portion 212 and the base portion 214 are vacuum molded as a single unit 600, joined together along one edge with a living hinge 620 separating the two. When the conjoined parts are folded along the living hinge 620 such that the convex surface 214A of the base portion 214 comes to lie against the concave surface 212A of the upper portion 212, the two parts fit together perfectly creating a longitudinal suction chamber between the two which is open at one end and closed at the other.

Further, affixing the bottom side 212A of the upper portion 212 to the upper side 214A of the base portion 214 can be accomplished by conventional adhesive or welding means. However, in a preferred embodiment they are affixed by the use of one or more plastic studs 630 and 640 which are molded into the plastic and spaced in such a way as to fit together when the upper portion 212 and the base portion 214 are caused to approximate each other by being folded along the living hinge 620. Use of the plastic studs 630 and 640 obviates the need for adhesives and greatly facilitates assembly of the apparatus 210.

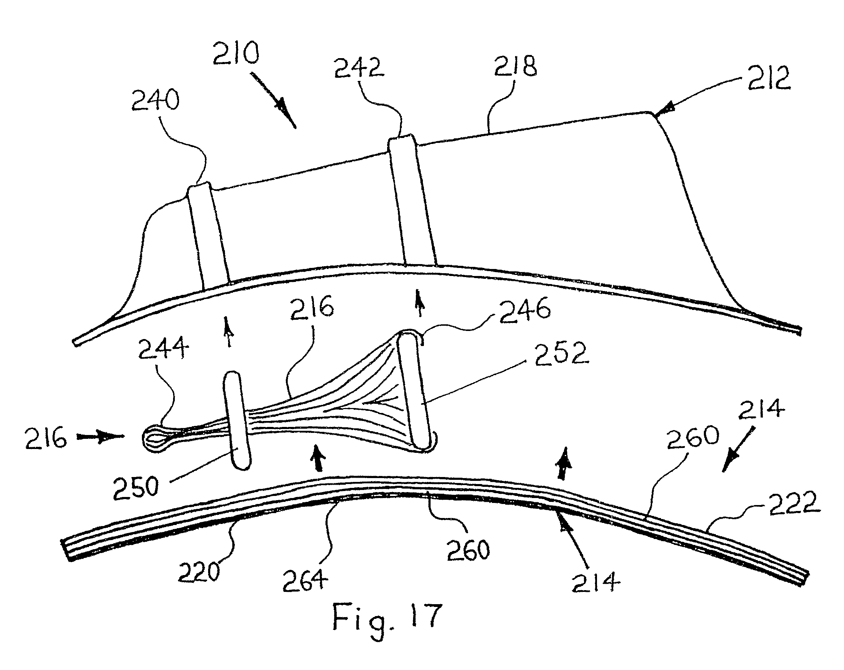

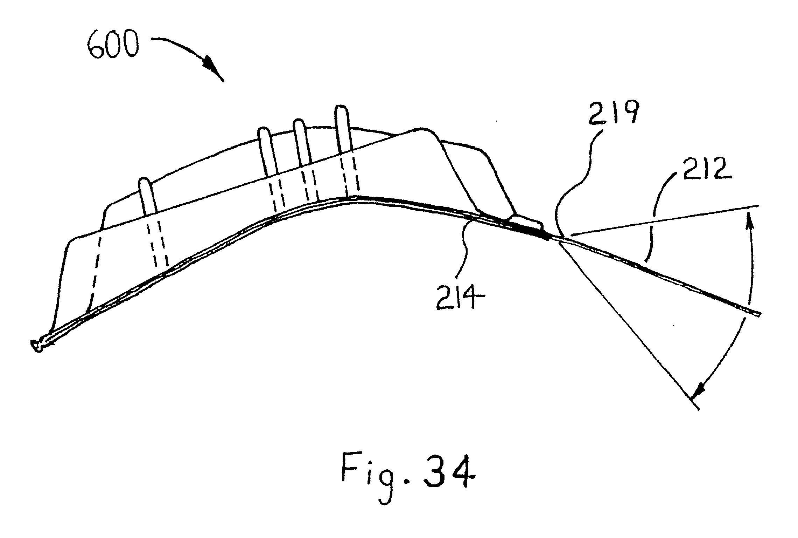

Fig. 34 illustrates the addition of a flexible extension to the semi-rigid apparatus to increase the conformity of the apparatus to different human body shapes. As set forth in Figure 17, the base portion 214 forms a generally rectangular shaped body, which has a contoured support surface 220. The lower side 224 of the base portion 214 is provided with pressure sensitive adhesive material 260 thereon and covered by a peel-off cover 264 which can be peeled off so that the adhesive material 260 can be securely attached to a surgical drape around a patient during surgery. The upper portion 212 is contoured to match the contoured support surface 220 of the base portion 214, which support surface 220 is contoured to generally match the curved shape of the human body. The base portion 214 or the upper portion 212 is each flexible when manufactured from thin plastic. When the upper portion 212 is affixed to the base portion 214 the resulting laminate is somewhat rigid and will not flex to match the different contours of different human body sizes.

In this embodiment, either the upper portion 212 or the base portion 214 is extended as illustrated in Figure 34 so that when the base portion and the upper portion are affixed to each other forming a rigid laminate, the extended segment 219 is not laminated and thus remains flexible. The resulting combination of a semi-rigid laminated surface and a flexible non-laminated surface will thus have added versatility in matching different human body sizes and shapes.

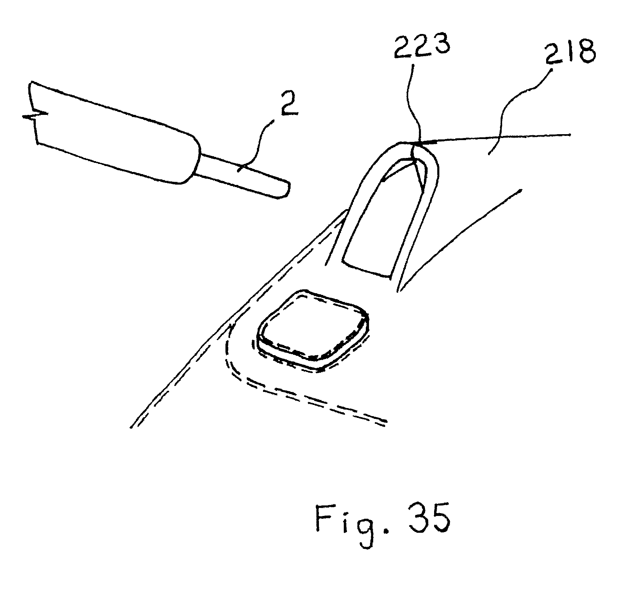

Referring to Figure 35, the present inventor has also conceived an improvement to remove debris from the cautery. The upside down channel 218 has a slit 223 formed therein at its front end. The cautery 2 often has debris formed thereon. In this improvement, the cautery 2 is run through the slit 223 and the debris is therefore scraped off the cautery and removed from the cautery so that it continues to function at an optimal condition.

An embodiment of the present invention is a shutoff valve apparatus used in conjunction with a suction device for stopping the suction action and muffling the noise from a suction nozzle of the suction device during surgery, the apparatus comprising: (a) a base portion having a contoured support surface with an upper side and a lower side, the lower side having means for securing and attaching the contoured support surface to a stationary object; (b) an upper portion having an elongated channel and a bottom side affixed to said upper side of said base portion to form a suction chamber with a closed end and an open end; (c) a flexible member having a first closed end and a second open end; (d) means for securely retaining said flexible member within said suction chamber to stop the suction action and muffle the noise from said suction nozzle when said suction device is within said flexible member; and (e) when said suction nozzle of such suction device is inserted into and through said open end of said suction chamber and into said flexible member through said second opened end, said suction nozzle pulls said flexible member toward itself so that the said flexible member stops the suction action of said suction nozzle and muffles the noise from said suction nozzle of said suction device and simultaneously grips said suction device within said suction chamber.

Of course the present invention is not intended to be restricted to any particular form or arrangement, or any specific embodiment, or any specific use, disclosed herein, since the same may be modified in various particulars or relations without departing from the spirit or scope of the claimed invention hereinabove shown and described of which the apparatus or method shown is intended only for illustration and disclosure of an operative embodiment and not to show all of the various forms or modifications in which this invention might be embodied or operated.

## Claims

1. A shutoff valve apparatus used in conjunction with a suction device for stopping the suction action and muffling the noise from a suction nozzle of the suction device during surgery, the apparatus comprising:
a. a base portion having a contoured support surface with an upper side and a lower side, the lower side having means for securing and attaching the contoured support surface to a stationary object;
b. an upper portion having an elongated channel and a bottom side affixed to said upper side of said base portion to form a suction chamber with a closed end and an open end;
c. a flexible member having a first closed end and a second open end; and
d. means for securely retaining said flexible member within said suction chamber to stop the suction action and muffle the noise from said suction nozzle when said suction device is within said flexible member; and
e. when said suction nozzle of such suction device is inserted into and through said open end of said suction chamber and into said flexible member through said second opened end, said suction nozzle pulls said flexible member toward itself so that the said flexible member stops the suction action of said suction nozzle and muffles the noise from said suction nozzle of said suction device and simultaneously grips said suction device within said suction chamber.

2. The apparatus in accordance with claim 1 wherein said retaining means includes a first retaining member attached to one of at least two spaced apart transverse grooves of said longitudinal suction chamber for securing and holding said first end of said flexible member, and a second retaining member attached to the other one of the at least two spaced apart transverse grooves of said longitudinal suction chamber for securing and holding said second end of said flexible member.

3. The apparatus in accordance with claim 2, wherein said flexible member contains a first closed end and an interior retaining member is inserted into the first closed end, the first retaining member including at least a surface with an opening so that the flexible member is inserted through the opening and the interior retaining member is retained on one side of the surface remote from the second retaining member.

4. The apparatus in accordance with claim 3, wherein said first end of the flexible member is retained within an additional sub-chamber within the suction chamber.

5. The apparatus in accordance with any one of claims 2 to 4, wherein said second retaining member includes a surface having a multiplicity of slits with a central opening, such that the flexible member is inserted through the opening in the surface and placed over the surface to retain the second open end of the flexible member within the suction chamber.

6. The apparatus in accordance with any preceding claim, wherein said suction device includes a suction hose and the apparatus includes means for retaining the suction hose to the apparatus.

7. The apparatus in accordance with claim 6, wherein said means for retaining the suction hose to the apparatus includes a hose clamping mechanism.

8. The apparatus in accordance with claim 7, wherein the hose clamping mechanism includes a multiplicity of spaced apart ribs.

9. The apparatus in accordance with any preceding claim, wherein said suction device includes a cautery and said apparatus includes means to enable the cautery to be cleaned.

10. The apparatus in accordance with any preceding claim, further comprising flexible means to enable the apparatus to conform to various shapes of the stationary object to which it is attached.

11. The apparatus in accordance with any preceding claim, further comprising the apparatus containing an upper portion and a lower portion and molded together by a living hinge which separates the upper and lower portion and means to enable the two parts of the apparatus to be attached together at a location remote from the living hinge.

12. The apparatus in accordance with any preceding claim, further comprising clamping means located adjacent to said suction chamber for clamping a suction hose of said suction device to said apparatus.

13. The apparatus in accordance with any preceding claim, wherein said upper portion has an elongated U-shaped upside down channel which forms the elongated channel when the upper portion is affixed to the upper side of the base portion to form the suction chamber.

14. The apparatus in accordance with any preceding claim, wherein the apparatus is made of polyethylene terephthalate (PET) material.

15. The apparatus in accordance with any preceding claim, wherein the means for securing and attaching the contour support surface to a stationary object is pressure sensitive adhesive which is covered with a peel-off cover tape.
